# EUROPÄISCHE PATENTSCHRIFT

(11) **EP 0 754 678 B1**
(45) Veröffentlichungstag und Bekanntmachung des Hinweises auf die Patenterteilung: **03.01.2001**
(21) Anmeldenummer: 96110967.5
(22) Anmeldetag: 08.07.1996
(51) Int. Cl.: C07C 229/48, A61K 31/195, C07K 5/023

(54) **Verbesserung der Verträglichkeit von pharmazeutisch wirksamen Beta-Aminosäuren**
Improvement of physiological tolerance of pharmaceutical active beta-amino acids
Amélioration de la tolérance physiologique d'acides aminés bêta cyclopentane

(30) Priorität: 19.07.1995 DE 19526274
(43) Veröffentlichungstag der Anmeldung: 22.01.1997
(60) Teilanmeldung: 00110958.6
(73) Patentinhaber: BAYER AG, 51368 Leverkusen (DE)
(72) Erfinder: Matzke, Michael, Dr., 42113 Wuppertal (DE); Militzer, Hans-Christian, Dr., 51467 Bergisch Gladbach (DE); Mittendorf, Joachim, Dr., 42113 Wuppertal (DE); Kunisch, Franz, Dr., 51519 Odenthal (DE); Schmidt, Axel, Dr., 42327 Wuppertal (DE); Schönfeld, Wolfgang, Dr., 42113 Wuppertal (DE); Ziegelbauer, Karl, Dr., 42115 Wuppertal (DE)

(56) Entgegenhaltungen:
- EP-A- 0 571 870
- WO-A-95/07022
- DE-A- 19 548 825
- DE-A- 19 604 225
- DATABASE WPI Section Ch, Week 9033 Derwent Publications Ltd., London, GB; Class B05, AN 90-250724 XP002042215 & JP 02 174 753 A (FUJISAWA PHARM CO LTD) , 6.Juli 1990
- OHKI HIDENORI ET AL.: "Synthesis and antifungal activity of FR 109615 Analogs" J. ANTIBIOT., Bd. 44, Nr. 5, 1991, Seiten 546-549, XP002042039

## Beschreibung

Aus den Publikationen EP-A-571 870, DOS 43 02 155, JP 021 747 53 A2 und J. Antibiot. (1991), 44 (5), 546-9 sind Cyclopentan- und -penten-β-aminosäuren bekannt. Solche β-Aminosäureverbindungen wirken antimikrobiell, insbesondere antimykotisch. Sie sind jedoch nicht frei von Nebenwirkungen.

Überraschenderweise wurde nun gefunden, dass Gemische aus α-Aminosäuren und Cyclopentan-β-aminosäuren aus der Gruppe bestehend aus "(S)-Isoleucin mit 2-Amino-4-methylencyclopentan-1-carbonsäure, (S)-Alanin mit 2-Amino-4-methylencyclopentan-1-carbonsäure, (S)-Prolin mit 1,2-cis-Aminocyclopentan-1-carbonsäure" oder isomere Formen davon diese unerwünschten Nebenwirkungen nicht oder nur in geringem Ausmaß aufweisen und damit eine verbesserte Verträglichkeit bei Warmblütern erreicht wird.

Beispielhaft für derartige erfindungsgemäße Gemische seien genannt: Gemisch aus (S)-Isoleucin mit 2-Amino-4-methylencyclopentan-1-carbonsäure, (S)-Alanin mit 2-Amino-4-methylen-cyclopentan-1-carbonsäure oder (S)-Prolin mit 1,2-cis-Amino-cyclopentan-1-carbonsäure.

Das molare Mischungsverhältnis von α-Aminosäure zu Cyclopentan-β-aminosäure im Bereich von 1:99 bis 99:1, bevorzugt 1:10 bis 10:1, besonders bevorzugt 1:5 bis 5:1 und ganz besonders bevorzugt 1:3 bis 3:1.

Die erfindungsgemäßen Gemische werden üblicherweise durch Mischen der bevorzugt fein pulverisierten Einzelkomponenten erhalten.

Die erfindungsgemäßen Gemische können aus im wesentlichen reinen Stereoisomeren oder Stereoisomerengemische bestehen.

Die vorstehend beschriebenen α-Aminosäuren und Cyclopentan-β-aminosäuren können auch in Form ihrer Salze vorliegen. Im allgemeinen seien hier Salze mit organischen oder anorganischen Basen oder Säuren sowie innere Salze genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifünktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin, Ethylendiamin oder Phenethylamin.

Die erfindungsgemäßen Gemische können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Ausgangspunkt für die vorliegende Erfindung war die Aufklärung des folgenden Mechanismus:

Die hier beschriebenen Cyclopentan-β-aminosäuren werden von verschiedenen Hefespecies durch Aminosäuretransporter akkumuliert. Der Transport der β-Aminosäuren kann durch aliphatische Aminosäuren, insbesondere, L-Leucin und L-Alanin, gehemmt werden. β-Aminosäuren hemmen die Proteinbiosynthese. Diese Hemmung kann durch eine der aliphatischen Aminosäuren, insbesondere durch L-Alanin, antagonisiert werden. Die gleichzeitige Gabe von β-Aminosäure und der antagonisierenden natürlichen Aminosäure als Gemisch führt zur Verringerung der bei Warmblütern auftretenden Nebenwirkungen bei gleichzeitiger Erhaltung der antimykotischen Wirkung in vivo.

Die erfindungsgemäßen Gemische zeigen daher ein nicht vorhersehbares, wertvolles pharmakologisches Wirkspektrum.

Die erfindungsgemäßen Gemische und ihre Säureadditions-Salze weisen in vivo antimikrobielle, insbesondere starke antimykotische Wirkungen auf. Gleichzeitig bedingt ihre geringere Toxizität eine bessere Verträglichkeit. Sie besitzen ein breites antimykotisches Wirkspektrum gegen Dermatophyten, wie Trichophyton mentagrophytes und Microsporum canis, gegen Sproßpilze wie Candida albicans, Candida glabrata, Epidermophyton floccosum und gegen Schimmelpilze wie Aspergillus niger und Aspergillus fumigatus. Die Aufzählung dieser Mikroorganismen stellt keinesfalls eine Beschränkung der bekämpfbaren Keime dar, sondern hat nur erläuternden Charakter. Sie sind deshalb geeignet zur Behandlung von Dermatomykosen und Systemmykosen.

### Prüfung der In-vivo-Wirksamkeit

Als Testmodell für antimykotische In-vivo-Wirkungen wurde die systemische Mäuse-Candidose benutzt:
Männliche CFW₁-Mäuse von 20 g Gewicht wurden durch Injektion von 3 x 10⁵ KBE von C. albicans pro Tier in die Schwanzvene infiziert.

Unbehandelte Kontrolltiere sterben vollständig innerhalb einer Woche post infectionem (p.i.) an einer generalisierten Candidose mit Granulombildung in der Niere. Zur Wirksamkeitsprüfung wurden die Präparate, gelöst in einer 0,2%igen wäßrigen Glucose-Agar-Lösung, den infizierten Tieren 2 mal täglich oral per Schlundsonde appliziert.

Die Tagesdosen betrugen 2 x 25 mg/kg und 2 x 50 mg/kg Körpergewicht (KG), die Therapiedauer betrug 5 Tage.

Die Überlebensraten der behandelten Tiere wurden täglich bis zum 10. Tag p.i. notiert. Zu diesem Zeitpunkt überlebten bei den unbehandelten Kontrolltieren keine Tiere.

Für die Präparate wurden pro Dosis- und Kontrollgruppe je 10 Tiere eingesetzt.

Alternativ kann die In-vivo-Wirksamkeit auch an Wistar-Ratten getestet werden. Diese würden geringere Tagesdosen, bezogen auf mg/kg KG, benötigen, um einen vergleichbaren Therapieeffekt zu erzielen. Der Test wird in diesem Fall wie folgt durchgeführt:

Acht Wochen alte, spezifisch pathogenfreie, männliche Wistar-Ratten von 200 g werden mit 5 x 10⁶ KBE Candida albicans in 0,5 ml PBS über die laterale Schwanzvene infiziert. Dieses führt zu einer 100 %igen Mortalität innerhalb von acht Tagen. Die Tiere zeigen schon einen Tag nach Infektion Blutungen im medialen Augenwinkel; neben der Niere werden andere Organsysteme wie Hirn, Herz, Leber, Milz, Retina und Lunge befallen. Substanzapplikation erfolgt zweimal täglich über fünf Tage peroral in je 1 ml Glucose (5%)-Agar (0,2%)-Lösung beginnend am Tag der Infektion.

Die bessere Verträglichkeit der erfindungsgemäßen Gemische wurde auf die folgende Weise getestet:

Wistar-Ratten wurden täglich mit den entsprechenden Substanzen gefüttert und der Gewichtsverlauf protokolliert. Es wurde entweder die β-Aminosäure alleine oder eine äquimolare Menge des entsprechenden Gemisches mit einer α-Aminosäure verabreicht. Nach 5 Tagen Behandlungsdauer war das Körpergewicht der Ratten bei Gabe der erfindungsgemäßen Gemische gleich geblieben oder leicht gestiegen, während es bei der Behandlung mit der β-Aminosäure um etwa 5 bis 10 % abgenommen hatte.

Zur vorliegenden Erfindung gehören auch Arzneimittel, enthaltend die erfindungsgemäßen Gemische sowie nicht-toxische, inerte pharmazeutische Träger- und Hilfsstoffe zur Bekämpfung von Krankheiten, insbesondere von Mykosen.

Der oder die Wirkstoffe können gegebenenfalls in einem oder mehreren der oben angegebenen Trägerstoffe auch in mikroverkapselter Form vorliegen.

Als bevorzugte pharmazeutische Zubereitungen seien Tabletten, Dragees, Kapseln. Pillen, Granulate, Suppositorien, Lösungen, Suspensionen und Emulsionen, Pasten, Salben, Gele, Cremes, Lotions, Puder und Sprays genannt.

Die therapeutisch wirksamen Gemische sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Wirkstoffe oder die Arzneimittel können oral und parenteral, appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Bei den erfindungsgemäßen Arzneimitteln handelt es sich üblicherweise um Kombinationspräparate zur gleichzeitigen, getrennten oder zeitlich abgestuften Anwendung bei der Bekämpfung von Krankheiten.

Bei Kombinationspräparaten zur gleichzeitigen Anwendung handelt es sich um Erzeugnisse, in denen die Einzelkomponenten der erfindungsgemäßen Gemische als physikalische Mischung vorliegen. Hierzu zählen insbesondere Tabletten, Dragees, Kapseln, Pillen, Suppositorien und Ampullen. Ebenso denkbar ist der Einsatz derartiger Mischungen als Lösung, Suspension oder Emulsion. Zu den Einzelkomponenten der erfindungsgemäßen Gemische zählen zum einen die α-Aminosäuren nachfolgend α-Aminosäurekomponente genannt und zum anderen die Cyclopentan-β-aminosäuren nachfolgend β-Aminosäurekomponente genannt.

Bei Kombinationspräparaten zur getrennten Anwendung handelt es sich um Erzeugnisse, in denen die Einzelkomponenten der erfindungsgemäßen Gemische räumlich voneinander getrennt vorliegen. Hierzu eignen sich insbesondere Tabletten, Dragees, Kapseln, Pillen und Suppositorien, die dieser Anforderung gerecht werden.

Ebenso denkbar sind Kombinationspräparate zur zeitlich abgestuften Anwendung. Diese ermöglichen die Verabreichung der Einzelkomponenten der erfindungsgemäßen Gemische in zeitlich versetzter Reihenfolge. Bei der zeitlich abgestuften Anwendung derartiger Kombinationspräparate ist es denkbar, die α-Aminosäurekomponente in bestimmter Weise im Verhältnis zur Verabreichung der β-Aminosäurekomponente zu verabreichen.Die α-Aminosäurekomponente kann dabei in der gleichen oder in einer anderen üblichen Applikationsform als die β-Aminosäurekomponente verabreicht werden, z.B. kann die β-Aminosäurekomponente intravenös, die α-Aminosäurekomponente dagegen peroral oder intravenös verabreicht werden.

Bei der zeitlich abgestuften Anwendung kann auch so vorgegangen werden, daß nur eine Teildosis der α-Aminosäurekomponente in der vorgenannten zeitlichen Relation zur Verabreichung der β-Aminosäurekomponente und die Restmenge der Gesamtdosis der α-Aminosäurekomponente in einer oder mehreren Teildosen innerhalb eines bestimmten Zeitraumes nach Verabreichung der β-Aminosäurekomponente verabreicht werden.

### Beispiel 1

(1R,2S)-2-Amino-4-methylen-cyclopentan-1-carbonsäure x (S)-Isoleucin 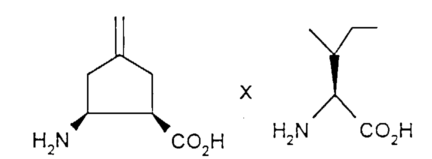 (-)-(1R,2S)-2-Amino-4-methylen-cyclopentan-1-carbonsäure (25,0 g, 177 mmol) und (S)-Isoleucin (23,2 g, 177 mmol) werden in Wasser (250 ml) und Ethanol (100 ml) in der Siedehitze gelöst. Man läßt die Lösung auf Raumtemperatur abkühlen und destilliert die Lösungsmittel im Vakuum bei 60°C ab. Ausbeute: 48,2 g (100 % d. Th.)
Schmp.: 230°C (Zers.)
¹H-NMR CDCl₃): δ = 0,95 (t, 3H), 1,00 (d, 3H), 1,18 - 1,35, 1,40- 1,52 (2m, 2H), 1,99 (cm, 1H), 2,52 - 2,67, 2,73 - 2,88 (2m, 4H); 3,09 (cm, 1H); 3,69 (d, 1H); 3,88 (cm, 1H); 5,09 (cm, 2H).
C₁₃H₂₄N₂O₄ (272,3)

### Beispiel 2

(-)-(1R,2S)-2-Amino-4-methylen-cyclopentan-1-carbonsäure (14,1 g, 100 mmol) und (S)-Isoleucin (26,2 g, 200 mmol) werden fein pulverisiert und anschließend pulverförmig gemischt.

### Beispiel 3

Eine Mischung aus (-)-(1R,2S)-2-Amino-4-methylen-cyclopentan-1-carbonsäure (14,1 g, 100 mmol) und (S)-Isoleucin (65,5 g, 500 mmol) wird analog zur Vorschrift des Beispiels 4 hergestellt.

## Patentansprüche

1. Antimikrobiell wirksame Gemische, enthaltend α-Aminosäuren und Cyclopentan-β-aminosäuren aus der Gruppe bestehend aus "(S)-lsoleucin mit 2-Amino-4-methylencyclopentan-1-carbonsäure, (S)-Alanin mit 2-Amino-4-methylencyclopentan-1-carbonsäure, (S)-Prolin mit 1,2-cis-Aminocyclopentan-1-carbonsäure" oder isomere Formen davon und gegebenenfalls einen oder mehrere pharmazeutisch annehmbare Trägerstoffe.

2. Gemische nach Anspruch 1 zur Verwendung bei der Behandlung von Krankheiten, insbesondere von Mykosen.

3. Arzneimittel, enthaltend Gemische nach Anspruch 1 zur Verwendung bei der Bekämpfung von Krankheiten, insbesondere von Mykosen.

4. Verwendung von Gemischen nach Anspruch 1 zur Herstellung von Arzneimitteln.

## Claims

1. Antimicrobially active mixtures, comprising α-amino acids and cyclopentane-β-amino acids from the group consisting of "(S)-isoleucine with 2-amino-4-methylenecyclopentane-1-carboxylic acid, (S)-alanine with 2-amino-4-methylenecyclopentane-1-carboxylic acid, (S)-proline with 1,2-cis-aminocyclopentane-1-carboxylic acid" or isomeric forms thereof and, if appropriate, one or more pharmaceutically acceptable excipients.

2. Mixtures according to Claim 1 for use in the treatment of diseases, in particular mycoses.

3. Medicaments comprising mixtures according to Claim 1 for use in the control of diseases, in particular mycoses.

4. Use of mixtures according to Claim 1 for preparing medicaments.

## Revendications

1. Mélanges doués d'activité antimicrobienne, contenant des α-aminoacides et des cyclopentane-β-aminoacides du groupe consistant en "la (S)-isoleucine avec l'acide 2-amino-4-méthylènecyclopentane-1-carboxylique, la (S)-alanine avec l'acide 2-amino-4-méthylènecyclopentane-1-carboxylique, la (S)-proline avec l'acide 1,2-cis-aminocyclopentane-1-carboxylique" ou leurs formes isomères et, le cas échéant, un ou plusieurs supports acceptables du point de vue pharmaceutique.

2. Mélanges suivant la revendication 1 destinés à être utilisés dans le traitement de maladies, notamment de mycoses.

3. Médicaments contenant des mélanges suivant la revendication 1, destinés à être utilisés pour combattre des maladies, en particulier des mycoses.

4. Utilisation de mélanges suivant la revendication 1 pour la préparation de médicaments.
